(19) **Europäisches Patentamt / European Patent Office / Office européen des brevets**

(11) **EP 4 491 173 A1**

(12) **EUROPEAN PATENT APPLICATION**
published in accordance with Art. 153(4) EPC

(43) Date of publication:
**15.01.2025 Bulletin 2025/03**

(21) Application number: 23767122.7

(22) Date of filing: **07.03.2023**

(51) International Patent Classification (IPC):
**A61K 9/16** (2006.01)  **A61K 38/08** (2019.01)

(52) Cooperative Patent Classification (CPC):
**A61K 9/16; A61K 38/08**

(86) International application number:
**PCT/KR2023/003080**

(87) International publication number:
**WO 2023/172021 (14.09.2023 Gazette 2023/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.03.2022 KR 20220029607**

(71) Applicant: **Dong Kook Pharm. Co., Ltd**
**Seoul 06072 (KR)**

(72) Inventors:
• **KIM, Kwang Jong**
**Yongin-si Gyeonggi-do 16822 (KR)**

• **KANG, Soo Yeon**
**Seoul 06721 (KR)**
• **OH, In Hyeok**
**Yongin-si Gyeonggi-do 16989 (KR)**
• **KIM, Byung Hyuck**
**Suwon-si Gyeonggi-do 16671 (KR)**
• **HAN, Su Min**
**Suwon-si Gyeonggi-do 16509 (KR)**
• **KIM, Keon Il**
**Suwon-si Gyeonggi-do 16521 (KR)**

(74) Representative: **Louis Pöhlau Lohrentz**
**Patentanwälte**
**Postfach 30 55**
**90014 Nürnberg (DE)**

(54) **METHOD FOR PREPARING MICROSPHERE WITH IMPROVED SUSPENSION PERFORMANCE, USING COACERVATION**

(57) The present invention discloses a method for preparing microspheres with improved suspension performance, using a coacervation method.

FIG. 1

Crude solution preparation — S1
Phase separation — S2
Hardening — S3
Washing — S4
Recovery — S5
Freeze-drying — S6

EP 4 491 173 A1

## Description

### Technical Field

The present invention relates to a method of producing microspheres with improved suspendability using a coacervation method, and more specifically, to a production method capable of significantly improving the suspendability and injectability of microspheres by a washing process using a surfactant.

### Background Art

Biodegradable microspheres are a dosage form developed for the purpose of long-term release of drugs *in vivo,* and may deliver drugs by subcutaneous injection or intramuscular injection.

Here, low suspendability and injectability of microspheres may reduce patient compliance because the microspheres clog the needle of the syringe upon *in vivo* injection or need to be injected using a larger syringe.

In general, the injectability of microspheres may be improved by making the particle size of the microspheres uniform or reducing the average particle size thereof.

However, this method has problems in that the physicochemical properties of the microspheres may vary depending on the particle size or distribution and the production method is complicated.

[Prior Art Documents]

[Patent Documents]

(Patent Document 1) European Patent Publication No. 0058481
(Patent Document 2) European Patent Publication No. 0092918

### DISCLOSURE

Technical Problem

An object of the present invention is to provide a method of producing microspheres using a coacervation method, which does not affect the physicochemical properties of drug-containing microspheres, uses a simple process, and may improve the water suspendability and injectability of microspheres.

Specifically, an object of the present invention is to provide a method of producing microspheres with improved suspendability using a coacervation method, and more specifically, an object of the present invention is to provide a method comprising a process of washing with an aqueous solution containing a surfactant.

Furthermore, a specific object of the present invention is to provide the method wherein the process of washing with an aqueous solution containing a surfactant such as polyvinyl alcohol or poloxamer is a continuous process without exposure to an external environment.

In addition, a special object of the present invention is to provide a method of producing microspheres using a coacervation method, which is capable of significantly improving the suspendability of microspheres in a solvent for injection and the injectability thereof by comprising a washing process.

### Technical Solution

To achieve the above objects, the present invention discloses the following means.

In one aspect, the present invention discloses a method of producing microspheres with improved suspendability using a coacervation method, comprising steps of: (S1) preparing a crude solution by adding and dispersing a second solution containing a pharmaceutical compound dissolved therein to a first solution containing a biocompatible polymer carrier substance dissolved therein; (S2) inducing the formation of microspheres by adding a phase inducer to the dispersion obtained in step (S1) to cause phase separation; (S3) hardening the microspheres by adding an emulsion to the mixture obtained in step (S2); (S4) washing the hardened microspheres of step (S3) with an aqueous solution containing a surfactant; (S5) recovering the washed microspheres of step (S4); and (S6) freeze-drying the recovered microspheres of step (S5).

**Advantageous Effects**

In the production method according to the present invention, the washing process using a surfactant is a continuous process without exposure to an external environment during the coacervation process and has the advantage of being able to be simply added and used.

In addition, the production method according to the present invention comprises a process of washing with an aqueous solution containing a surfactant after the hardening step, and thus may increase the wettability of the surface of the microspheres, thereby effectively improving the suspendability and injectability of the microspheres, unlike a process without a washing step.

Therefore, when the microspheres produced by the production method according to the present invention are used as an injection agent, they have the effect of improving patient compliance due to a reduced needle size resulting from the improved injectability thereof compared to commercial products.

The effects of the present invention are not limited to the effects mentioned above, and may various effects within a range obvious to those skilled in the art from the following description.

**Brief Description of Drawings**

FIG. 1 is a flow chart showing a method of producing microspheres with improved suspendability using a coacervation method according to the present invention.

FIG. 2 depicts photographs showing the results of SEM analysis performed to analyze the morphology of microspheres produced in Examples 2 to 4.

FIG. 3 depicts photographs showing the results of SEM analysis performed to analyze the morphology of microspheres produced in Examples 5 to 7.

FIGS. 4 to 6 depict graphs showing the results of *in vitro* drug release evaluation of octreotide for microspheres (unwashed formulation) produced in Comparative Example 1, microspheres (formulation washed with PVA) produced in Examples 2 to 4, and microspheres (formulation washed with poloxamer 188) produced in Examples 5 to 7.

FIG. 7 is a graph showing the results of an injection force test for microspheres according to Examples 2 to 4.

FIG. 8 is a graph showing the results of an injection force test for microspheres according to Examples 5 to 7.

FIG. 9 depicts photographs showing the results of an injectability test for microspheres according to Comparative Example 1 and Example 1.

FIG. 10 depicts photographs showing the results of a suspension test for microspheres according to Comparative Example 1 and Example 1.

FIG. 11 is a graph showing the blood concentration profile obtained after administering microspheres produced according to Comparative Example 1 and Example 1 to rats.

**Best Mode**

Hereinafter, the present specification will be described in more detail.

This is explained in detail as follows. The terms used in the present specification are currently widely used general terms selected in consideration of their functions in the present invention, but they may change depending on the intents of those skilled in the art, precedents, or the advents of new technology. Additionally, in certain cases, there may be terms arbitrarily selected by the applicant, and in this case, their meanings are described in a corresponding description part of the present invention. Accordingly, terms used in the present invention should be defined based on the meaning of the term and the entire contents of the present invention, rather than the simple term name.

Unless otherwise defined, all terms used herein, including technical or scientific terms, have the same meanings as understood by those skilled in the art to which the present invention pertains. Terms such as those used in general and defined in dictionaries should be interpreted as having meanings identical to those specified in the context of related technology. Unless definitely defined in the present application, the terms should not be interpreted as having ideal or excessively formative meanings.

A numerical range includes numerical values defined in the range. Every maximum numerical limitation given throughout the present specification includes every lower numerical limitation, as if such lower numerical limitations were expressly written herein. Every minimum numerical limitation given throughout the present specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Furthermore, each description and embodiment disclosed in the present invention may also be applied to other descriptions and embodiments. That is, all combinations of the various components disclosed in the present invention fall within the scope of the present invention. In addition, the scope of the present invention cannot be construed as being limited by the specific descriptions described below.

As used herein, terms such as "containing" or "comprising" should be understood as open-ended terms implying the possibility of including other embodiments.

In order to achieve the above-described objects, the present inventors have conducted research and development on a method of producing drug-encapsulated microspheres using a coacervation method. As a result, the present inventors have found that, when a washing process using an aqueous solution containing a surfactant is additionally performed, it is possible to improve the suspendability and injectability of microspheres by increasing the surface wettability of the microspheres, unlike a process without a washing process, and accordingly, when the microspheres are used as an injection agent, it is possible improve patient compliance due to a reduced needle size resulting from the improved injectability of the microspheres compared to a commercial product (Sandostatin, 19-gauge), thereby completing the present invention.

Hereinafter, the present invention will be described in detail.

## Method of producing microspheres with improved suspendability using coacervation method

To achieve the above-described objects, the present invention discloses the following means.

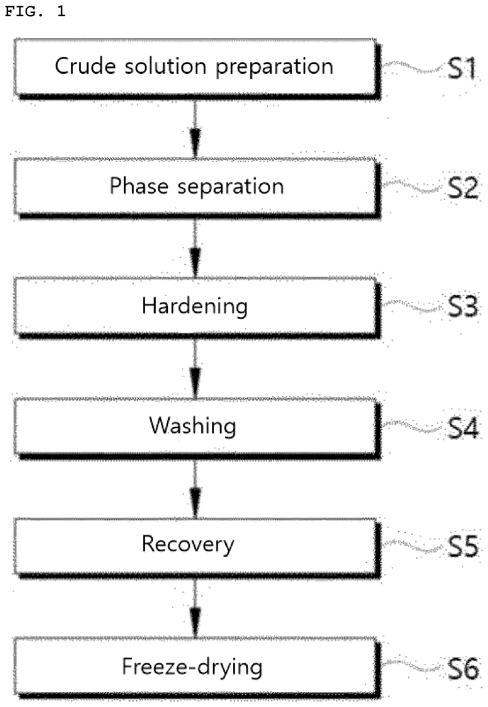

Referring to FIG. 1, in one aspect, the present invention provides a method of producing microspheres with improved suspendability using a coacervation method, comprising steps of: (S1) preparing a crude solution by adding and dispersing a second solution containing a pharmaceutical compound dissolved therein to a first solution containing a biocompatible polymer carrier substance dissolved therein; (S2) inducing the formation of microspheres by adding a phase inducer to the dispersion obtained in step (S1) to cause phase separation; (S3) hardening the microspheres by adding an emulsion to the mixture obtained in step (S2); (S4) washing the hardened microspheres of step (S3) with an aqueous solution containing a surfactant; (S5) recovering the washed microspheres of step (S4); and (S6) freeze-drying the recovered microspheres of step (S5).

In the present invention, the term "coacervation method" refers to a method of producing microspheres by separating a concentrated colloidal sol from a dispersion medium by a decrease in hydration of colloidal particles in a hydrophilic colloidal solution and an electrostatic factor.

In the present invention, the first solution in step (S1) is prepared using a first solvent capable of dissolving only the biocompatible polymer carrier substance without dissolving the pharmaceutical compound, and the first solvent in the first solution may be methylene chloride, without being limited thereto.

In the present invention, the second solution in step (S1) is prepared using a second solvent capable of dissolving only the pharmaceutical compound without dissolving the biocompatible polymer carrier substance, and the second solvent in the second solution may be methanol, without being limited thereto.

In the present invention, the biocompatible polymer carrier substance in step (S1) may be any one or more selected from the group consisting of polylactic acid (PLA), polylactic-co-glycolic acid (PLGA), polyphosphazene, poly(imino carbonate), polyphosphoester, polyanhydride, polyorthoester, a copolymer of lactic acid and caprolactone, polycaprolactone, polyhydroxyvalerate, polyhydroxybutyrate, polyamino acid, a copolymer of lactic acid and amino acid, and mixtures thereof, without being limited thereto.

In the present invention, the pharmaceutical compound in step (S1) may be a peptide drug, wherein the peptide drug may be octreotide or a salt thereof, wherein the salt of octreotide may be octreotide acetate, without being limited thereto.

In the present invention, the phase inducer in step (S2) may be silicone oil, without being limited thereto.

In the present invention, the emulsion in step (S3) may be an oil-in-water (O/W) type emulsion, and may include heptane, phosphate buffer (pH 4), silicone oil, and sorbitan oleate, without being limited thereto.

In the present invention, the surfactant in step (S4) may be a nonionic surfactant, and may be any one or more selected from the group consisting of polysorbate, poloxamer, polyethylene glycol, and polyvinyl alcohol. Specifically, the surfactant may be poloxamer or polyvinyl alcohol, without being limited thereto.

In the present invention, the concentration of the surfactant in step (S4) may be 0.05 to 7 wt% based on the total weight of the aqueous solution. Specifically, the concentration of the surfactant may be 0.1 to 5 wt% based on the total weight of the aqueous solution, without being limited thereto.

Specifically, when a washing process is performed with an aqueous solution containing the surfactant at a concentration within the above range, there are advantages in that it is possible to the suspendability and injectability of drug-encapsulated microspheres, and it is possible to improve the appearance of the produced microspheres by increasing the yield.

In the present invention, the washing process in step (S4) may be a continuous process without exposure to an external environment during a coacervation process, without being limited thereto.

## Microspheres produced using coacervation method

In one aspect, the present invention provides microspheres produced by the above-described production method.

In the present invention, the average particle size of the microspheres may be 30 to 50 um, without being limited thereto.

In the present invention, the span value of the microspheres may be 1.55 or less. Specifically, when the microspheres are washed with an aqueous solution containing polyvinyl alcohol, the span value of the microspheres may be 1.10 to 1.34, and when the microspheres are washed with an aqueous solution containing poloxamer, the span value of the microspheres may be 1.14 to 1.55, without being limited thereto.

In the present invention, the pharmaceutical compound contained in the microsphere may be encapsulated in an amount of 10 to 30 mg, without being limited thereto.

In the present invention, the dissolution pattern of the microspheres containing the pharmaceutical compound, measured using a dissolution solution containing acetonitrile at pH 10, may satisfy all of the following conditions, without being limited thereto:

1) the dissolution rate of the pharmaceutical compound at a dissolution time of 1 hour is 10.0%; and
2) the dissolution rate of the pharmaceutical compound at a dissolution time of 24 hours is 60% to 90%.

## Pharmaceutical composition for parenteral administration comprising microspheres

In one aspect, the present invention provides a pharmaceutical composition for parenteral administration comprising the microspheres.

In the present invention, the term "carrier" refers to a compound that facilitates the addition of a compound into a cell or tissue, and the term "pharmaceutically acceptable" refers to a composition which is physiologically acceptable and, when administered to humans, does not cause allergic reactions such as gastrointestinal disorders and dizziness, or similar reactions.

In one embodiment of the present invention, the pharmaceutically acceptable carrier is one commonly used for formulation, and examples thereof include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil.

In the present invention, the term "parenteral administration" refers to administration by injection, excluding oral administration. Specifically, the route of administration by injection may be any of subcutaneous injection, intradermal injection, or intramuscular injection, and the dosage form may be appropriately selected depending on the intended use of the composition.

In addition, the pharmaceutical composition for parenteral administration according to the present invention relates to a pharmaceutical composition for the prevention or treatment of acromegaly, gastrointestinal and pancreatic endocrine tumors, or progressive neuroendocrine tumors, and may reduce the needle size (23- to 26-gauge) compared to a conventional commercially available product (Sandostatin, 19-gauge), thereby improving patient compliance.

In the present invention, the term "prevention" means delaying the onset of acromegaly, gastrointestinal and pancreatic endocrine tumors, or progressive neuroendocrine tumors. If the onset of acromegaly, gastrointestinal and pancreatic endocrine tumors, or progressive neuroendocrine tumors is delayed for an expected period of time, prevention may be considered complete.

In the present invention, the term "treatment" means partially or completely mitigating, ameliorating, alleviating, inhibiting or delaying the onset of acromegaly, gastrointestinal or pancreatic endocrine tumors, or progressive neuroendocrine tumors, thereby reducing the severity or reducing incidence of one or more symptoms or features.

The matters mentioned with respect to the production method of the present invention, the microspheres produced thereby, and the parenteral pharmaceutical composition comprising the same are applied equally unless they are mutually contradictory.

Hereinafter, the present invention will be described in detail by way of examples. However, the following examples are only intended to illustrate the present invention, and the scope of the present invention is not limited thereto.

[Examples]

**Example 1. Production of Octreotide Acetate-Encapsulated Microspheres Using Coacervation Method (Washing Process Using Polyvinyl Alcohol)**

About 3.8 g of a linear or branched polymer (50/50 mol, $M_w$=47,000) consisting of poly(D,L-lactide-glycolide) was dissolved in 43 mL of methylene chloride, and then 224.6 mg of octreotide acetate (200.5 mg as octreotide) dissolved in about 0.9 mL of methanol was added to the solution, thereby preparing a peptide-polymer mixed solvent. About 60 mL of silicone oil (polydimethylsiloxane, Dow 360 Medical Fluid, 1000 cs) was added to the peptide-polymer mixed solvent at an appropriate rate. The resulting mixture was added to a stirred emulsion containing 1,200 mL of n-heptane, 270 mL of phosphate buffer (pH 4), 35 mL of silicone oil (polydimethylsiloxane, Dow 360 Medical Fluid, 350 cs), and 2 mL of the emulsifier sorbitan oleate (Span 80®). Microspheres were produced by continued stirring at an appropriate stirring speed for a predetermined period of time.

The produced microspheres were recovered by vacuum filtration, and then stirred for at least 30 minutes in an aqueous solution containing 0.5 wt% (w/w) of polyvinyl alcohol (PVA, Sigma-Aldrich, Lot.no: P8136, product name: Poly(vinyl alcohol) 87-90% hydrolyzed, average mol wt 30,000-70,000) (the concentration of PVA is 0.5 wt% based on the total weight of the aqueous solution) dissolved therein and recovered. The recovered microspheres were freeze-dried overnight and then obtained (yield: 71.3%, average particle size: 31.4 μm).

**Example 2. Production of Octreotide Acetate-Encapsulated Microspheres Using Coacervation Method (Washing Process Using Polyvinyl Alcohol)**

Octreotide acetate-encapsulated microspheres were produced under the same conditions as in Example 1 (yield: 63.5%, average particle size: 34.1 pm), except that an "aqueous solution containing 0.1 wt% of polyvinyl alcohol (PVA) (the concentration of PVA is 0.1 wt% based on the total weight of the aqueous solution)" dissolved therein was used instead of the "aqueous solution containing 0.5 wt% of polyvinyl alcohol (PVA) dissolved therein".

**Example 3. Production of Octreotide Acetate-Encapsulated Microspheres Using Coacervation Method (Washing Process Using Polyvinyl Alcohol)**

Octreotide acetate-encapsulated microspheres were produced under the same conditions as in Example 1 (yield: 60.4%, average particle size: 32.5 pm), except that an "aqueous solution containing 1.0 wt% of polyvinyl alcohol (PVA) (the concentration of PVA is 1.0 wt% based on the total weight of the aqueous solution)" dissolved therein was used instead of the "aqueous solution containing 0.5 wt% of polyvinyl alcohol (PVA) dissolved therein".

**Example 4. Production of Octreotide Acetate-Encapsulated Microspheres Using Coacervation Method (Washing Process Using Polyvinyl Alcohol)**

Octreotide acetate-encapsulated microspheres were produced under the same conditions as in Example 1 (yield: 57.1%, average particle size: 30.8 pm), except that an "aqueous solution containing 5.0 wt% of polyvinyl alcohol (PVA) (the concentration of PVA is 5.0 wt% based on the total weight of the aqueous solution)" dissolved therein was used instead of the "aqueous solution containing 0.5 wt% of polyvinyl alcohol (PVA) dissolved therein".

**Example 5. Production of Octreotide Acetate-Encapsulated Microspheres Using Coacervation Method (Washing Process Using Poloxamer 188 (P188)))**

About 3.8 g of a linear or branched polymer (50/50 mol, $M_w$=47,000) consisting of poly(D,L-lactide-glycolide) was dissolved in 43 mL of methylene chloride, and then 224.6 mg of octreotide acetate (200.5 mg as octreotide) dissolved in about 0.9 mL of methanol was added to the solution, thereby preparing a peptide-polymer mixed solvent. About 60 mL of silicone oil (Dow 360 Medical Fluid, 1000 cs) was added to the peptide-polymer mixed solvent at an appropriate rate. The resulting mixture was added to a stirred emulsion containing 1,200 mL of n-heptane, 270 mL of phosphate buffer (pH 4), 35 mL of silicone oil (polydimethylsiloxane, Dow 360 Medical Fluid, 350 cs), and 2 mL of the emulsifier sorbitan oleate (Span 80®). Microspheres were produced by continued stirring at an appropriate stirring speed for a predetermined period of time.

The produced microspheres were recovered by vacuum filtration, and then stirred for at least 30 minutes in an aqueous solution containing 0.1 wt% (w/w) of Poloxamer 188 (average molecular weight: 8,905 g/mol) (the concentration of Poloxamer 188 is 0.1 wt% based on the total weight of the aqueous solution) dissolved therein and recovered. The recovered microspheres were freeze-dried overnight and then obtained (yield: 54.3%, average particle size: 34.2 μm).

**Example 6. Production of Octreotide Acetate-Encapsulated Microspheres Using Coacervation Method (Washing Process Using Poloxamer 188 (P188)))**

Octreotide acetate-encapsulated microspheres were produced under the same conditions as in Example 5 (yield: 61.7%, average particle size: 32.2 $\mu$m), except that an "aqueous solution containing 1.0 wt% of Poloxamer 188 (the concentration of Poloxamer 188 is 1.0 wt% based on the total weight of the aqueous solution)" dissolved therein was used instead of the "aqueous solution containing 0.1 wt% of Poloxamer 188 dissolved therein".

**Example 7. Production of Octreotide Acetate-Encapsulated Microspheres Using Coacervation Method (Washing Process Using Poloxamer 188 (P188)))**

Octreotide acetate-encapsulated microspheres were produced under the same conditions as in Example 5 (yield: 63.3%, average particle size: 33.8 pm), except that an "aqueous solution containing 5.0 wt% of Poloxamer 188 (the concentration of Poloxamer 188 is 5.0 wt% based on the total weight of the aqueous solution)" dissolved therein was used instead of the "aqueous solution containing 0.1 wt% of Poloxamer 188 dissolved therein".

**Comparative Example 1. Production of Octreotide Acetate-Encapsulated Microspheres Using Coacervation Method (without Washing Process)**

About 3.8 g of a linear or branched polymer (50/50 mol, $M_w$=47,000) consisting of poly(D,L-lactide-glycolide) was dissolved in 43 mL of methylene chloride, and then 224.6 mg of octreotide acetate (200.5 mg as octreotide) dissolved in about 0.9 mL of methanol was added to the solution, thereby preparing a peptide-polymer mixed solvent. About 60 mL of silicone oil (Dow 360 Medical Fluid, 1000 cs) was added to the peptide-polymer mixed solvent at an appropriate rate. The resulting mixture was added to a stirred emulsion containing 1,200 mL of n-heptane, 270 mL of phosphate buffer (pH 4), 35 mL of silicone oil (polydimethylsiloxane, Dow 360 Medical Fluid, 350 cs), and 2 mL of the emulsifier sorbitan oleate (Span 80®). Microspheres were produced by continued stirring at an appropriate stirring speed for a predetermined period of time.
The produced microspheres were recovered by vacuum filtration and then freeze-dried overnight and then obtained (yield: 30.5%, average particle size: 49.0 $\mu$m).

**Comparative Example 2. Production of Octreotide Acetate-Encapsulated Microspheres Using Coacervation Method (Washing Process Using Distilled Water)**

About 3.8 g of a linear or branched polymer (50/50 mol, $M_w$=47,000) consisting of poly(D,L-lactide-glycolide) was dissolved in 43 mL of methylene chloride, and then 224.6 mg of octreotide acetate (200.5 mg as octreotide) dissolved in about 0.9 mL of methanol was added to the solution, thereby preparing a peptide-polymer mixed solvent. About 60 mL of silicone oil (polydimethylsiloxane, Dow 360 Medical Fluid, 1000 cs) was added to the peptide-polymer mixed solvent at an appropriate rate. The resulting mixture was added to a stirred emulsion containing 1,200 mL of n-heptane, 270 mL of phosphate buffer (pH 4), 35 mL of silicone oil (polydimethylsiloxane, Dow 360 Medical Fluid, 350 cs), and 2 mL of the emulsifier sorbitan oleate (Span 80®). Microspheres were produced by continued stirring at an appropriate stirring speed for a predetermined period of time.
The produced microspheres were recovered by vacuum filtration, and then stirred for at least 30 minutes in 1,200 mL of distilled water and recovered. The recovered microspheres were freeze-dried overnight and then obtained (yield: 40.7%, average particle size: 33.6 $\mu$m).

[Experimental Examples]

**Experimental Example 1. Microsphere Size Measurement and Morphology Evaluation**

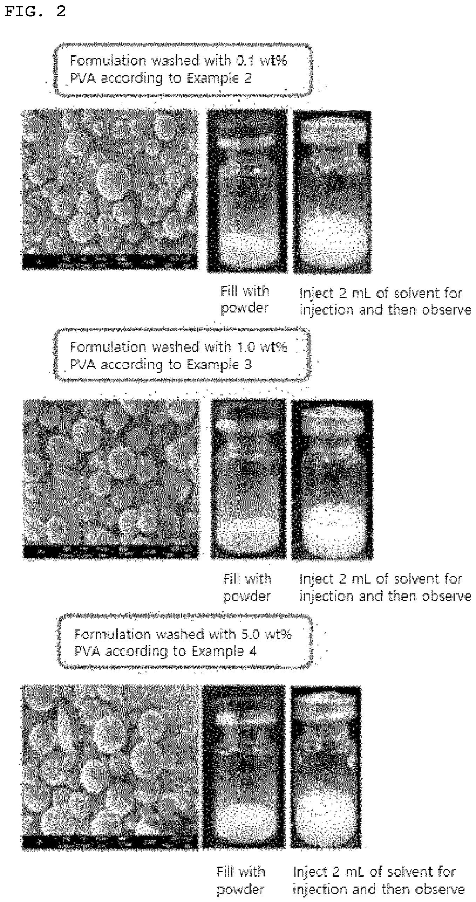

The particle size distributions of the microspheres produced in Examples 2 to 4 were analyzed using a particle size analyzer (Malvern-MASTERSLZER 3000), and the results are shown in Table 1 below. In addition, the morphology of the microspheres was analyzed using a scanning electron microscope (SEM, EmCRAFTS-CUBE II), and the results are shown in FIG. 2.
Referring to Table 1, the $d_{10}$, $d_{50}$, and $d_{90}$ of the microspheres produced according to Example 2 were measured as 19.8, 34.1, and 63.2 um, respectively, the $d_{10}$, $d_{50}$, and $d_{90}$ of the microspheres produced according to Example 3 were measured as 18.6, 32.5, and 62.0 $\mu$m, respectively, and the $d_{10}$, $d_{50}$, and $d_{90}$ of the microspheres produced according to Example 4 were measured as 18.4, 30.8, and 52.2 um, respectively. The span (meaning the width of particle size distribution) values calculated from these results using Equation 1 below were 1.28 (Example 2), 1.34 (Example 3), and 1.10 (Example 4).

[Equation 1]

$$\text{Span} = d_{90} - d_{10} / d_{50}$$

The span values were measured to be less than 2.0, suggesting that the microspheres produced according to Examples 2 to 4 have a uniform size. In addition, it was shown that the microspheres produced according to Examples 2 to 4 have a size that can be administered *in vivo* by intramuscular or subcutaneous injection.

In addition, referring to FIG. 2, it was confirmed that, as the microspheres produced in Examples 2 to 4 were subjected to the process of washing with the aqueous solution containing polyvinyl alcohol, the microspheres did not aggregate and had a uniform appearance.

From the above results, it could be confirmed that there was no significant difference in the physicochemical evaluation (particle size) between the microspheres according to Examples 2 to 4, suggesting that the above-described concentration range of polyvinyl alcohol (0.1 to 5%) is a range suitable for pharmaceutical quality.

[Table 1]

| | Formulation washed with 0.1% PVA according to Example 2 | Formulation washed with 1.0% PVA according to Example 3 | Formulation washed with 5.0% PVA according to Example 4 |
|---|---|---|---|
| Particle size (μm) $d_{10}/d_{50}/d_{90}$ | 19.8/34.1/63.2 | 18.6/32.5/62.0 | 18.4/30.8/52.2 |

(In Table 1 above, $d_{10}$, $d_{50}$ and $d_{90}$, which represent the sizes of the microspheres, mean particle sizes corresponding to 10%, 50% and 90% of the cumulative particle distribution, respectively.)

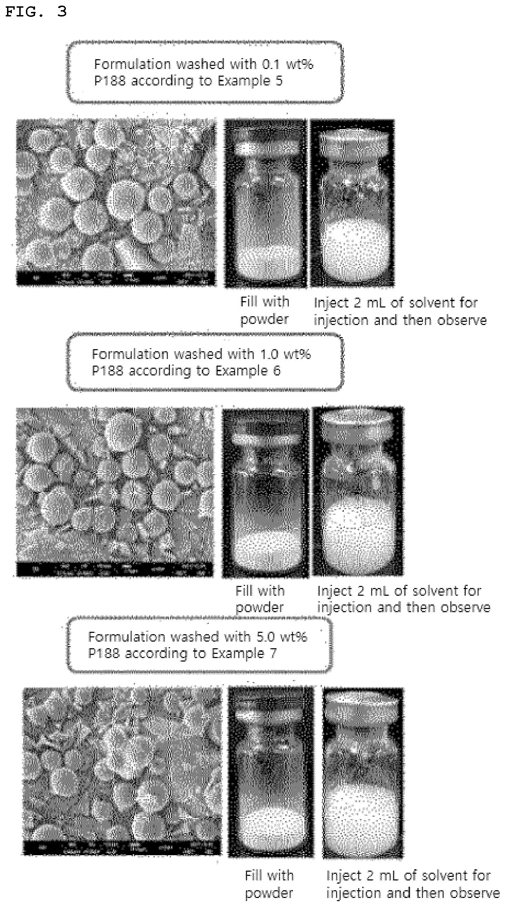

In addition, the particle size distributions and morphologies of the microspheres produced in Examples 5 to 7 were analyzed using the above-mentioned methods, and the results are shown in Table 2 below and FIG. 3.

Referring to Table 2 below, the $d_{10}$, $d_{50}$, and $d_{90}$ of the microspheres produced according to Example 5 were measured as 18.9, 34.2, and 71.8 um, respectively, the $d_{10}$, $d_{50}$, and $d_{90}$ of the microspheres produced according to Example 6 were measured as 19.1, 32.2, and 55.8 μm, respectively, and the $d_{10}$, $d_{50}$, and $d_{90}$ of the microspheres produced according to Example 7 were measured as 20.0, 33.8, and 58.8 um, respectively. The span (meaning the width of particle size distribution) values calculated using Equation 1 above were 1.55 (Example 5), 1.14 (Example 6), and 1.15 (Example 7).

The span values were measured to be less than 2.0, suggesting that the microspheres produced according to Examples 5 to 7 have a uniform size. In addition, it was shown that the microspheres produced according to Examples 5 to 7 have a size that can be administered *in vivo* by intramuscular or subcutaneous injection.

In addition, referring to FIG. 3, it was confirmed that, as the microspheres produced in Examples 5 to 7 were subjected to the process of washing with the aqueous solution containing Poloxamer 188, the microspheres did not aggregate and had a uniform appearance.

From the above results, it could be confirmed that there was no significant difference in the physicochemical evaluation (particle size) between the microspheres according to Examples 5 to 7, suggesting that the above-described concentration range of Poloxamer 188 (0.1 to 5%) is a range suitable for pharmaceutical quality.

[Table 2]

| | Formulation washed with 0.1% Poloxamer 188 according to Example 5 | Formulation washed with 1.0% Poloxamer 188 according to Example 6 | Formulation washed with 5.0% Poloxamer 188 according to Example 7 |
|---|---|---|---|
| Particle size (μm) $d_{10}/d_{50}/d_{90}$ | 18.9/34.2/71.8 | 19.1/32.2/55.8 | 20.0/33.8/58.8 |

(In Table 2 above, $d_{10}$, $d_{50}$ and $d_{90}$, which represent the sizes of the microspheres, mean particle sizes corresponding to 10%, 50% and 90% of the cumulative particle distribution, respectively.)

**Experimental Example 2. Evaluation of Octreotide Content in Microspheres**

The octreotide content in the microspheres produced in each of Examples 2 to 4 was evaluated using a high-performance liquid chromatography (HPLC) analysis method.

Specifically, about 60 mg of the microspheres produced in each of Examples 2 to 4 were placed in a 50 mL flask and completely dissolved in tetrahydrofuran (THF). Then, acetate buffer solution was added up to the marked line and the dissolved octreotide was extracted. Using the extract as the sample, the content of octreotide encapsulated in the microspheres (drug encapsulation rate) was measured using HPLC (instrument name: 1260 infinity LC, Agilent). The results are shown in Table 3 below.

Referring to Table 3, it could be confirmed that the microspheres according to Examples 2 to 4 had a suitable octreotide content and there was no significant difference in the physicochemical evaluation (content) between the microspheres according to Examples 2 to 4, suggesting that the above-described concentration range of polyvinyl alcohol (0.1 to 5%) is a range suitable for pharmaceutical quality.

[Table 3]

|  | Formulation washed with 0.1% PVA according to Example 2 | Formulation washed with 1.0% PVA according to Example 3 | Formulation washed with 5.0% PVA according to Example 4 |
|---|---|---|---|
| Content (%) | 102.67% | 102.85% | 107.06% |

In addition, the content of octreotide in the microspheres produced in each of Examples 5 to 7 was analyzed using the method mentioned above, and the results are shown in Table 4 below.

Referring to Table 4, it could be confirmed that the microspheres according to Examples 5 to 7 had a suitable octreotide content and there was no significant difference in the physicochemical evaluation (content) between the microspheres according to Examples 5 to 7, suggesting that the above-described concentration range of Poloxamer 188 (0.1 to 5%) is a range suitable for pharmaceutical quality.

[Table 4]

|  | Formulation washed with 0.1% Poloxamer 188 according to Example 5 | Formulation washed with 1.0% Poloxamer 188 according to Example 6 | Formulation washed with 5.0% Poloxamer 188 according to Example 7 |
|---|---|---|---|
| Content (%) | 97.97% | 98.10% | 102.93% |

## Experimental Example 3. In *Vitro* Drug Release Evaluation of Octreotide

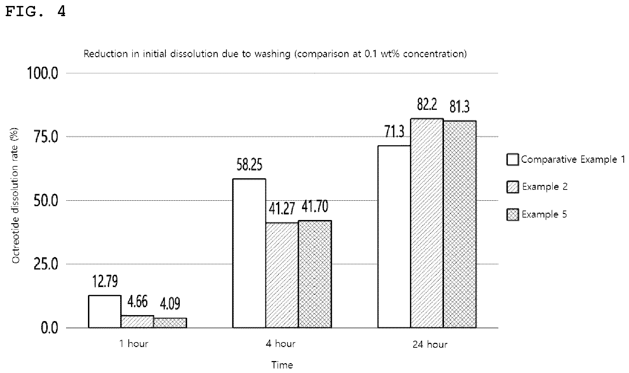

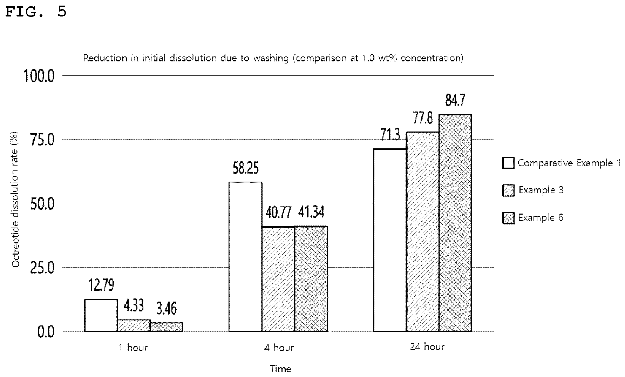

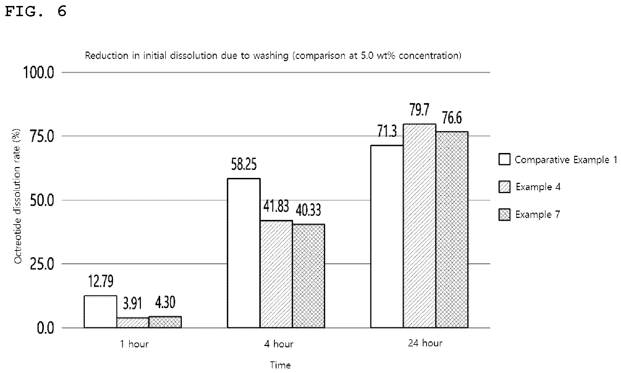

An *in vitro* release test was conducted for the microspheres (unwashed formulation) produced in Comparative Example 1, the microspheres (formulation washed with PVA) produced in Examples 2 to 4, and the microspheres (formulation washed with Poloxamer 188) produced in Examples 5 to 7. Each 30 mg of the freeze-dried microspheres were dispersed in an elution buffer (pH 10.0) and then subjected to a release test at a constant stirring speed at $37 \pm 0.5°C$. After sampling for each test time, the obtained microspheres were completely dissolved in tetrahydrofuran (THF), extracted with acetate buffer, and then analyzed by an HPLC analysis method. Here, as the analysis conditions, tetramethylammonium hydroxide/acetylcholine/distilled water (TMAH/ACH/DW) was used as the mobile phase, measurement was performed at 210 nm and a flow rate of 1.8 mL/min, and the dissolution rates at 1 hour, 4 hours, and 24 hours were evaluated. The results are shown in FIGS. 4 to 6.

Referring to FIGS. 4 to 6, it was confirmed that the microspheres (unwashed formulation) according to Comparative Example 1 satisfied the long-term dissolution standard (24-hour standard: dissolution of 58 to 90%), but exceeded the initial dissolution standard (1-hour standard: dissolution of less than 10%, 4-hour standard: dissolution of 10 to 500).

However, in the case of the microspheres (formulation washed with PVA) according to Examples 2 to 4 and the microspheres (formulation washed with Poloxamer 188) according to Examples 5 to 7, it could be confirmed that the initial dissolution could be suppressed and the long-term dissolution was also satisfied, because the washing process with the aqueous solution containing the surfactant was performed.

## Experimental Example 4. Injection Force Test

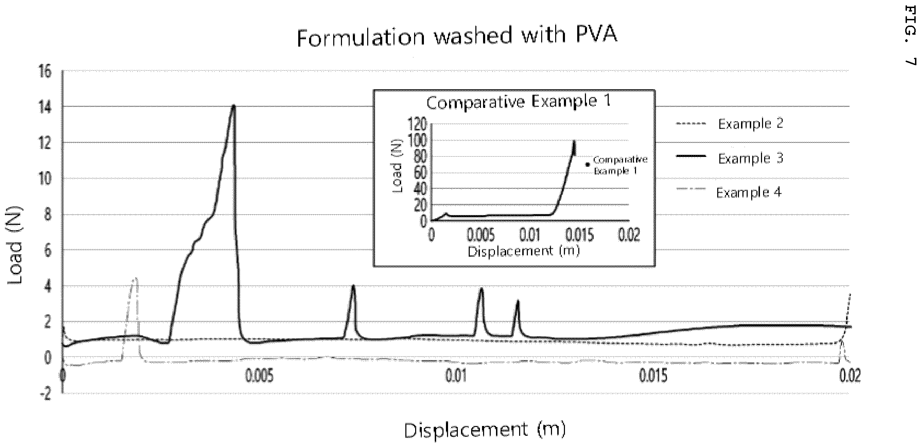

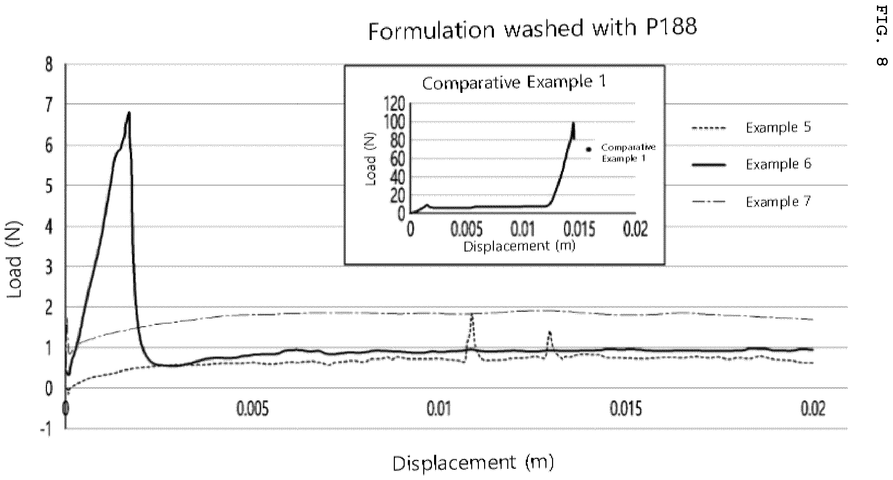

Each quantitative sample was suspended in a solvent for injection solvent through a syringe and 22-gauge and subjected to an injection force test, and the results are shown in FIGS. 7 and 8.

<Sample Information>

1) Unwashed formulation (Comparative Example 1)
2) Formulation washed with 0.1 wt% PVA (Example 2), formulation washed with 1.0 wt% PVA (Example 3), and formulation washed with 5.0 wt% PVA (Example 4)
3) Formulation washed with 0.1 wt% P188 (Example 5), formulation washed with 1.0 wt% P188 (Example 6), and formulation washed with 5.0 wt% P188 (Example 7)

(1) Visual results

It was confirmed that the formulations washed with PVA according to Examples 2 to 4 had good suspendability without the occurrence of bubbles in the solvent for injection within the vial. When injected into the syringe, the formulation washed with PVA was completely transferred into the syringe without any microspheres sticking to the vial wall.

(2) Results of injection force test

Referring to FIGS. 7 and 8, it was confirmed that the formulation washed with PVA (Examples 2 to 4) and the formulations washed with Poloxamer 188 (Examples 5 to 7) had improved injectability compared to the unwashed formulation (Comparative Example 1).
In addition, it was confirmed that, in the case of the formulation washed with 1.0 wt% PVA, pressure was applied initially, but the overall injection pressure was significantly lower than that of the unwashed formulation.

**Experimental Example 5. Injectability Test**

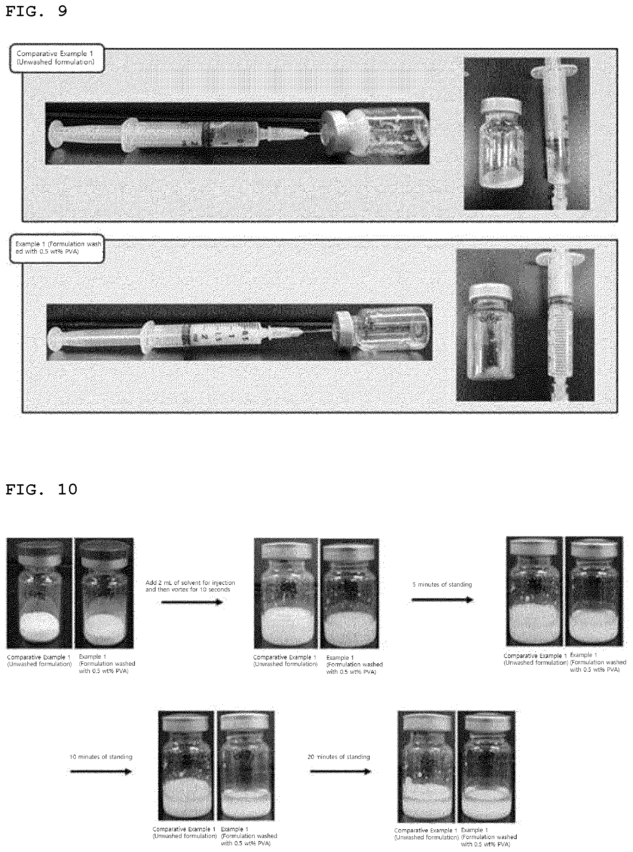

The microspheres (unwashed formulation) according to Comparative Example 1 and the microspheres (formulation washed with 0.5 wt% PVA) according to Example 1 were suspended in a solvent for injection using a 23-gauge 2 mL syringe and subjected to an injectability test, and the results are shown in FIG. 9.
Referring to FIG. 9, it was confirmed that the microspheres (unwashed formulation) according to Comparative Example 1 remained separated from the solvent for injection, and only the solvent for injection existed in the syringe.
However, it was confirmed that the microspheres (formulation washed with 0.5 wt% PVA) according to Example 1 were well suspended in the solvent for injection and were easily introduced into the syringe, suggesting that the injectability was significantly improved by performing the washing process with the aqueous solution containing PVA according to the Example.

**Experimental Example 6. Suspension Test**

About 625 mg of the microspheres (unwashed formulation) according to Comparative Example 1 and about 625 mg of the microspheres (formulation washed with 0.5 wt% PVA) according to Example 1 were each added to 2 mL of the solvent for injection, and then vortexed for 10 seconds. Thereafter, the appearance of each sample was observed while standing (standing times: 5 minutes, 10 minutes, and 20 minutes), and the results are shown in FIG. 10.
Referring to FIG. 10, it was confirmed that, in the case of the microspheres (unwashed formulation) according to Comparative Example 1, foaming occurred after 5 minutes of standing, and the appearance was bulky.
However, it was confirmed that, in the case of the microspheres (formulation washed with 0.5 wt% PVA) according to Example 1, no foaming occurred and the appearance was soft.
From the above results, it can be seen that the microspheres (formulation washed with 0.5 wt% PVA) according to Example 1 can be administered in a predetermined amount without foaming in the syringe.

**Experimental Example 7. Individual Variation in Blood Octreotide Concentration**

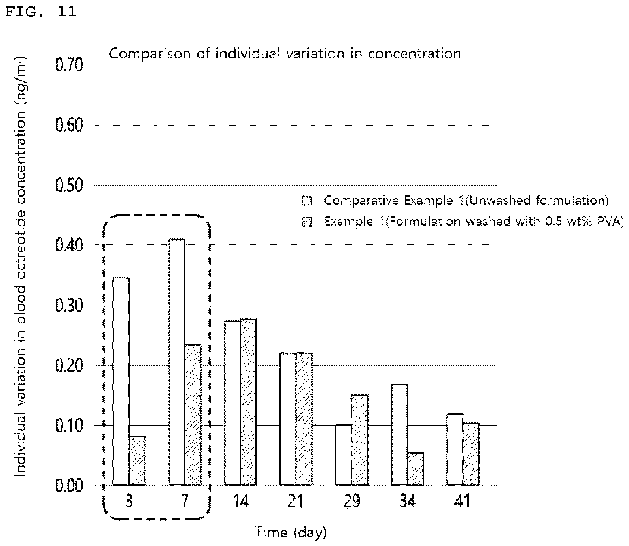

About 53 mg of the microspheres (unwashed formulation) according to Comparative Example 1 and about 53 mg of the microspheres (formulation washed with 0.5 wt% PVA) according to Example 1 were suspended in a vehicle and administered intramuscularly (I.M.) to rats at an octreotide dose of 10 mg/kg. About 0.5 mL of a blood sample was collected at predetermined time points, pretreated, and then analyzed for the blood octreotide concentration using LC/MS/MS. The results are shown in Table 5 below and FIG. 11.
Referring to Table 5 and FIG. 11, it was confirmed that, when the formulation washed with PVA was administered, individual variation was not improved at all the blood collection time points, but individual variation was reduced at specific time points (day 3 and day 7).

[Table 5]

| In vivo (time, day) | Comparative Example 1 | Example 1 |
|---|---|---|
| 3 | 0.35 | 0.08 |
| 7 | 0.41 | 0.24 |
| 14 | 0.28 | 0.28 |
| 21 | 0.22 | 0.22 |
| 29 | 0.10 | 0.15 |
| 34 | 0.17 | 0.06 |
| 41 | 0.12 | 0.10 |

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

**Claims**

1. A method of producing microspheres with improved suspendability using a coacervation method, comprising steps of:

(S1) preparing a crude solution by adding and dispersing a second solution containing a pharmaceutical compound dissolved therein to a first solution containing a biocompatible polymer carrier substance dissolved therein;
(S2) inducing formation of microspheres by adding a phase inducer to the dispersion obtained in step (S1) to cause phase separation;
(S3) hardening the microspheres by adding an emulsion to the mixture obtained in step (S2);
(S4) washing the hardened microspheres of step (S3) with an aqueous solution containing a surfactant;
(S5) recovering the washed microspheres of step (S4); and
(S6) freeze-drying the recovered microspheres of step (S5) .

2. The method of claim 1, wherein the biocompatible polymer carrier substance is any one or more selected from the group consisting of polylactic acid (PLA), polylactic-co-glycolic acid (PLGA), polyphosphazene, poly(imino carbonate), polyphosphoester, polyanhydride, polyorthoester, a copolymer of lactic acid and caprolactone, polycaprolactone, polyhydroxyvalerate, polyhydroxybutyrate, polyamino acid, a copolymer of lactic acid and amino acid, and mixtures thereof.

3. The method of claim 1, wherein the pharmaceutical compound is a peptide drug.

4. The method of claim 3, wherein the peptide drug is octreotide or a salt thereof.

5. The method of claim 1, wherein the surfactant is any one or more selected from the group consisting of polysorbate, poloxamer, polyethylene glycol, and polyvinyl alcohol.

6. The method of claim 1, wherein a concentration of the surfactant is 0.05 to 7 wt% based on the total weight of the aqueous solution.

FIG. 1

```
┌─────────────────────────────────┐
│   Crude solution preparation    │ ── S1
└─────────────────────────────────┘
                  │
                  ▼
┌─────────────────────────────────┐
│        Phase separation         │ ── S2
└─────────────────────────────────┘
                  │
                  ▼
┌─────────────────────────────────┐
│           Hardening             │ ── S3
└─────────────────────────────────┘
                  │
                  ▼
┌─────────────────────────────────┐
│            Washing              │ ── S4
└─────────────────────────────────┘
                  │
                  ▼
┌─────────────────────────────────┐
│            Recovery             │ ── S5
└─────────────────────────────────┘
                  │
                  ▼
┌─────────────────────────────────┐
│          Freeze-drying          │ ── S6
└─────────────────────────────────┘
```

FIG. 2

Formulation washed with 0.1 wt%
PVA according to Example 2

Fill with    Inject 2 mL of solvent for
powder       injection and then observe

Formulation washed with 1.0 wt%
PVA according to Example 3

Fill with    Inject 2 mL of solvent for
powder       injection and then observe

Formulation washed with 5.0 wt%
PVA according to Example 4

Fill with    Inject 2 mL of solvent for
powder       injection and then observe

FIG. 3

Formulation washed with 0.1 wt% P188 according to Example 5

Fill with powder

Inject 2 mL of solvent for injection and then observe

Formulation washed with 1.0 wt% P188 according to Example 6

Fill with powder

Inject 2 mL of solvent for injection and then observe

Formulation washed with 5.0 wt% P188 according to Example 7

Fill with powder

Inject 2 mL of solvent for injection and then observe

EP 4 491 173 A1

FIG. 4

Reduction in initial dissolution due to washing (comparison at 0.1 wt% concentration)

Octreotide dissolution rate (%)

- 100.0
- 75.0
- 50.0
- 25.0
- 0.0

**1 hour**: 12.79, 4.66, 4.09
**4 hour**: 58.25, 41.27, 41.70
**24 hour**: 71.3, 82.2, 81.3

Time

Legend:
☐ Comparative Example 1
▨ Example 2
▨ Example 5

FIG. 5

FIG. 6

Reduction in initial dissolution due to washing (comparison at 5.0 wt% concentration)

FIG. 7

Formulation washed with PVA

FIG. 8

Formulation washed with P188

FIG. 9

FIG. 10

FIG. 11

Comparison of individual variation in concentration

Y-axis: Individual variation in blood octreotide concentration (ng/ml)

X-axis: Time (day) — 3, 7, 14, 21, 29, 34, 41

Legend:
□ Comparative Example 1(Unwashed formulation)
▨ Example 1(Formulation washed with 0.5 wt% PVA)

## INTERNATIONAL SEARCH REPORT

| International application No. |
|---|
| **PCT/KR2023/003080** |

| **A.** | **CLASSIFICATION OF SUBJECT MATTER** |
|---|---|

**A61K 9/16**(2006.01)i; **A61K 38/08**(2006.01)i

According to International Patent Classification (IPC) or to both national classification and IPC

| **B.** | **FIELDS SEARCHED** |
|---|---|

Minimum documentation searched (classification system followed by classification symbols)

A61K 9/16(2006.01); A61K 38/00(2006.01); A61K 38/02(2006.01); A61K 38/08(2006.01); A61K 38/09(2006.01); A61K 38/26(2006.01); A61K 9/14(2006.01); A61K 9/50(2006.01); A61K 9/52(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 코아세르베이션(coacervation), 미립구(microsphere), 세척(wash), 계면활성제 (surfactant), 옥트레오타이드(octreotide)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| Y | US 2011-0086104 A1 (RICKEY, M. E. et al.) 14 April 2011 (2011-04-14) See paragraphs [0073]-[0075]; and claims 1, 3, 4 and 7. | 1-6 |
| Y | US 2012-0082731 A1 (RAICHE, A. et al.) 05 April 2012 (2012-04-05) See paragraph [0016]; and claims 1, 6, 7 and 14. | 1-6 |
| A | US 2008-0317865 A1 (TRAPANI, K. et al.) 25 December 2008 (2008-12-25) See entire document. | 1-6 |
| A | US 2008-0233199 A1 (KUMAR, R. et al.) 25 September 2008 (2008-09-25) See entire document. | 1-6 |
| A | KR 10-2007-0031304 A (AMYLIN PHARMACEUTICALS, INC. et al.) 19 March 2007 (2007-03-19) See entire document. | 1-6 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 June 2023** | **08 June 2023** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/003080**

| Patent document cited in search report | | | Publication date (day/month/year) | Patent family member(s) | | | Publication date (day/month/year) |
|---|---|---|---|---|---|---|---|
| US | 2011-0086104 | A1 | 14 April 2011 | AT | 501711 | T | 15 April 2011 |
| | | | | AU | 2002-324780 | B2 | 03 May 2007 |
| | | | | CA | 2454027 | A1 | 13 March 2003 |
| | | | | CA | 2454027 | C | 15 April 2008 |
| | | | | CY | 1112348 | T1 | 09 December 2015 |
| | | | | DK | 1420762 | T3 | 27 June 2011 |
| | | | | EP | 1420762 | A1 | 26 May 2004 |
| | | | | EP | 1420762 | B1 | 16 March 2011 |
| | | | | EP | 2283820 | A1 | 16 February 2011 |
| | | | | ES | 2362865 | T3 | 14 July 2011 |
| | | | | HK | 1066465 | A1 | 24 March 2005 |
| | | | | IL | 160378 | A | 30 December 2010 |
| | | | | JP | 2002-097161 | A | 02 April 2002 |
| | | | | JP | 2005-502674 | A | 27 January 2005 |
| | | | | JP | 2011-037882 | A | 24 February 2011 |
| | | | | JP | 2013-216676 | A | 24 October 2013 |
| | | | | JP | 2015-227383 | A | 17 December 2015 |
| | | | | JP | 4035595 | B2 | 23 January 2008 |
| | | | | JP | 5841565 | B2 | 13 January 2016 |
| | | | | MX | PA04001765 | A | 22 November 2004 |
| | | | | NZ | 531165 | A | 26 August 2005 |
| | | | | PT | 1420762 | E | 27 June 2011 |
| | | | | US | 2002-0048540 | A1 | 25 April 2002 |
| | | | | US | 2003-0118660 | A1 | 26 June 2003 |
| | | | | US | 2005-0077169 | A1 | 14 April 2005 |
| | | | | US | 2005-0079224 | A1 | 14 April 2005 |
| | | | | US | 2006-0099271 | A1 | 11 May 2006 |
| | | | | US | 2007-0196499 | A1 | 23 August 2007 |
| | | | | US | 2008-0050447 | A1 | 28 February 2008 |
| | | | | US | 2008-0051561 | A1 | 28 February 2008 |
| | | | | US | 2009-0194894 | A1 | 06 August 2009 |
| | | | | US | 6824822 | B2 | 30 November 2004 |
| | | | | US | 7223440 | B2 | 29 May 2007 |
| | | | | US | 7524530 | B2 | 28 April 2009 |
| | | | | US | 7875310 | B2 | 25 January 2011 |
| | | | | US | 8187672 | B2 | 29 May 2012 |
| | | | | WO | 03-020245 | A1 | 13 March 2003 |
| | | | | WO | 03-020245 | A8 | 08 July 2004 |
| US | 2012-0082731 | A1 | 05 April 2012 | AU | 2011-308893 | A1 | 05 April 2012 |
| | | | | AU | 2011-308893 | A1 | 09 May 2013 |
| | | | | AU | 2011-308893 | B2 | 23 April 2015 |
| | | | | AU | 2011-308897 | A1 | 23 May 2013 |
| | | | | AU | 2011-308897 | A1 | 05 April 2012 |
| | | | | AU | 2011-308897 | B2 | 18 June 2015 |
| | | | | CA | 2813301 | A1 | 05 April 2012 |
| | | | | CA | 2813302 | A1 | 05 April 2012 |
| | | | | CN | 103298453 | A | 11 September 2013 |
| | | | | CN | 103370057 | A | 23 October 2013 |
| | | | | EP | 2621473 | A2 | 07 August 2013 |
| | | | | EP | 2621474 | A2 | 07 August 2013 |

Form PCT/ISA/210 (patent family annex) (July 2022)

**INTERNATIONAL SEARCH REPORT**
**Information on patent family members**

International application No.

**PCT/KR2023/003080**

| Patent document cited in search report | | | Publication date (day/month/year) | Patent family member(s) | | | Publication date (day/month/year) |
|---|---|---|---|---|---|---|---|
| | | | | IL | 225254 | A | 27 June 2013 |
| | | | | IL | 225255 | A | 27 June 2013 |
| | | | | JP | 2013-538854 | A | 17 October 2013 |
| | | | | JP | 2013-538855 | A | 17 October 2013 |
| | | | | JP | 5808413 | B2 | 10 November 2015 |
| | | | | RU | 2013119810 | A | 10 November 2014 |
| | | | | RU | 2013119811 | A | 10 November 2014 |
| | | | | US | 2012-0083444 | A1 | 05 April 2012 |
| | | | | WO | 2012-044671 | A2 | 05 April 2012 |
| | | | | WO | 2012-044671 | A3 | 14 March 2013 |
| | | | | WO | 2012-044675 | A2 | 05 April 2012 |
| | | | | WO | 2012-044675 | A3 | 16 August 2012 |
| US | 2008-0317865 | A1 | 25 December 2008 | WO | 2008-157540 | A1 | 24 December 2008 |
| US | 2008-0233199 | A1 | 25 September 2008 | AU | 2008-231093 | A1 | 02 October 2008 |
| | | | | CA | 2680365 | A1 | 02 October 2008 |
| | | | | EP | 2131815 | A1 | 16 December 2009 |
| | | | | JP | 2010-522196 | A | 01 July 2010 |
| | | | | US | 2012-0258914 | A1 | 11 October 2012 |
| | | | | WO | 2008-118712 | A1 | 02 October 2008 |
| KR | 10-2007-0031304 | A | 19 March 2007 | AU | 2005-235100 | A1 | 03 November 2005 |
| | | | | CA | 2560874 | A1 | 03 November 2005 |
| | | | | CA | 2560874 | C | 19 February 2013 |
| | | | | CA | 2798552 | A1 | 03 November 2005 |
| | | | | CN | 101065116 | A | 31 October 2007 |
| | | | | CN | 101065116 | B | 19 November 2014 |
| | | | | CN | 104382880 | A | 04 March 2015 |
| | | | | EP | 1734935 | A1 | 27 December 2006 |
| | | | | EP | 1958623 | A1 | 20 August 2008 |
| | | | | EP | 2133073 | A1 | 16 December 2009 |
| | | | | EP | 2366385 | A1 | 21 September 2011 |
| | | | | EP | 2821063 | A1 | 07 January 2015 |
| | | | | EP | 2821063 | B1 | 18 July 2018 |
| | | | | EP | 3466412 | A1 | 10 April 2019 |
| | | | | EP | 3466412 | B1 | 12 January 2022 |
| | | | | IL | 178335 | B | 27 February 2014 |
| | | | | IL | 230632 | A | 31 March 2014 |
| | | | | IL | 230632 | B | 29 November 2018 |
| | | | | JP | 2007-532682 | A | 15 November 2007 |
| | | | | JP | 2012-051930 | A | 15 March 2012 |
| | | | | JP | 4899021 | B2 | 21 March 2012 |
| | | | | US | 2005-0271702 | A1 | 08 December 2005 |
| | | | | US | 2006-0110423 | A1 | 25 May 2006 |
| | | | | US | 2008-0125348 | A1 | 29 May 2008 |
| | | | | US | 2008-0125349 | A1 | 29 May 2008 |
| | | | | US | 2009-0035253 | A1 | 05 February 2009 |
| | | | | US | 2010-0152097 | A1 | 17 June 2010 |
| | | | | US | 2010-0152111 | A1 | 17 June 2010 |
| | | | | US | 2014-0031281 | A1 | 30 January 2014 |
| | | | | US | 7456254 | B2 | 25 November 2008 |
| | | | | US | 7612176 | B2 | 03 November 2009 |

Form PCT/ISA/210 (patent family annex) (July 2022)

<table>
<tr><td colspan="2" rowspan="2"><strong>INTERNATIONAL SEARCH REPORT</strong><br>Information on patent family members</td><td colspan="2">International application No.</td></tr>
<tr><td colspan="2"><strong>PCT/KR2023/003080</strong></td></tr>
</table>

| Patent document<br>cited in search report | Publication date<br>(day/month/year) | Patent family member(s) | | Publication date<br>(day/month/year) |
|---|---|---|---|---|
| | | US 8293871 | B2 | 23 October 2012 |
| | | US 8431685 | B2 | 30 April 2013 |
| | | US 8461105 | B2 | 11 June 2013 |
| | | WO 2005-102293 | A1 | 03 November 2005 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0058481 A
- EP 0092918 A